# EUROPEAN PATENT SPECIFICATION

(11) **EP 2 043 700 B1**
(45) Date of publication and mention of the grant of the patent: **19.10.2011**
(21) Application number: 07777204.4
(22) Date of filing: 22.05.2007
(51) Int. Cl.: A61L 29/02, A61L 29/10, A61L 29/18, A61L 31/02, A61L 31/08, A61L 31/18

(54) **MEDICAL DEVICES HAVING A TEMPORARY RADIOPAQUE COATING**
MEDIZINPRODUKTE MIT EINER TEMPORÄREN RÖNTGENOPAKEN BESCHICHTUNG
DISPOSITIF MÉDICAL À REVÊTEMENT TEMPORAIREMENT RADIOOPAQUE

(30) Priority: 07.07.2006 US 481943
(43) Date of publication of application: 08.04.2009
(73) Proprietor: Boston Scientific Limited, Christ Church (BB)
(72) Inventor: CLARKE, John, T., County Galway (IE); O'BRIEN, Barry, Barna, Galway (IE)
(74) Representative: Lang, Johannes
(86) International application number: PCT/US2007/012142
(87) International publication number: WO 2008/008126

(56) References cited:
- EP-A- 0 894 503
- WO-A-01/49340
- US-A1- 2004 033 345
- US-A1- 2006 058 867

## Description

### TECHNICAL FIELD

The present invention relates to implantable medical devices having a radiopaque coating.

### BACKGROUND

Many medical devices are implanted inside the body with the aid of x-racy fluoroscopy, which provides real-time visualization of the implantation procedure. For example, vascular stents are typically implanted by a catheterization procedure using x-ray fluoroscopy to guide the stent through the vasculature and position it in the target artery. Thus, the stent and/or stent deployment system must be sufficiently radiopaque (not transparent to x-rays) for visualization under x-ray fluoroscopy.

Weeks or months after the implantation of a stent, a subsequent visualization of the stented artery is often necessary in order to diagnose possible reocclusion (restenosis) of the artery. Typically, the diagnosis of restenosis is made by repeating an invasive catheterization procedure in order to obtain an x-ray fluoroscopic image (angiogram) of the stented artery.

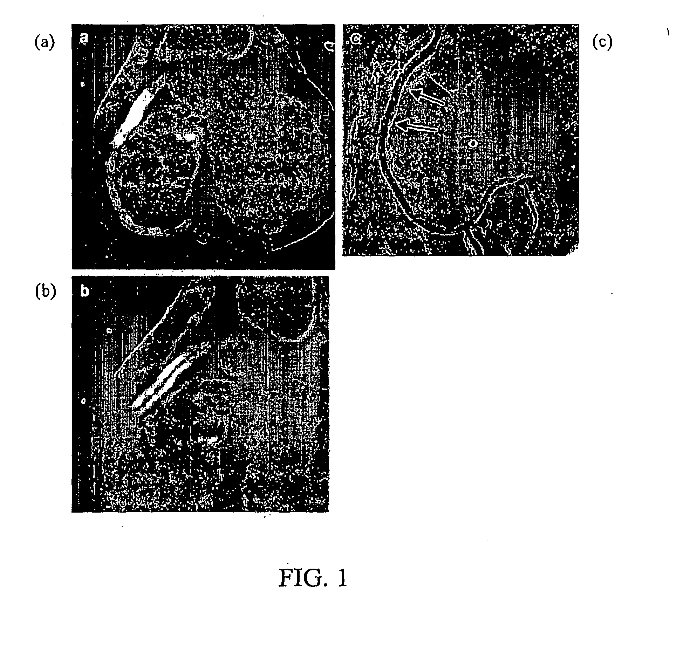

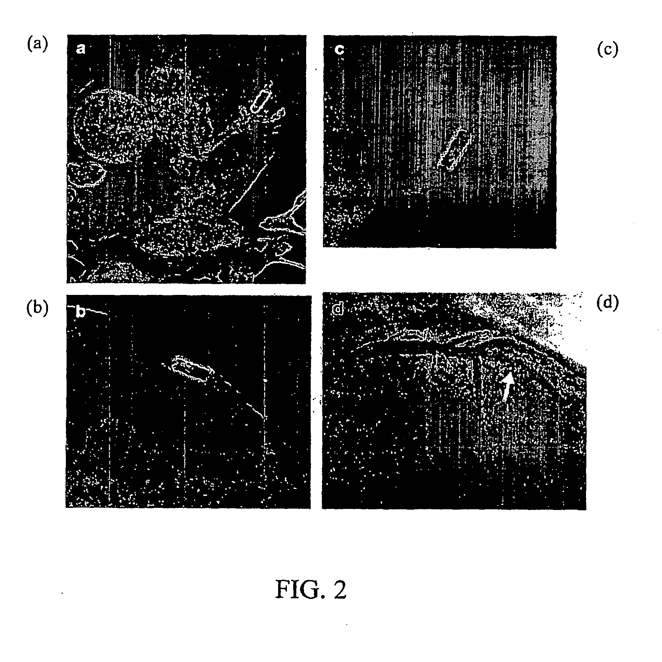

However, there is now growing interest in the non-invasive imaging of stented arteries by CT angiography using high resolution, multi-detector CT scanners as an alternative to catheterization and fluoroscopic imaging for the diagnosis of restenosis. But the use of CT angiography in this context has been limited because of distortions (artifacts) in the image caused by the metallic stent, which are sufficiently radiopaque for fluoroscopic visualization, but are often too radiopaque for high sensitivity CT imaging. For example, FIG. 1(c) is an angiogram of a stented artery obtained by x-ray fluoroscopy confirming that the stented artery (black arrows) is unobstructed. FIGS. 1(a) and 1(b) show CT angiography images of the same stented artery (black arrows). These images demonstrate the metallic artifacts, as characterized by the exaggerated thickening of the stent struts, which obscure the lumen of the stented artery. FIGS.2 (a)-(d) show a set of similar images demonstrating this phenomenon.

US 2006/0058867 A1 describes an elastomeric radiopaque adhesive composition of a biocompatible elastomeric matrix and a radiopaque material distributed therein. EP-A-0 894 503 describes a temporary bioabsorbable radiopaque marker for use on an implantable prosthesis. WO 01/49340 A describes a stent having a coating of radiopaque particles contained in a polymeric binder.

### SUMMARY OF THE INVENTION

There is a need for a medical device such as a stent that is temporarily radiopaque for imaging under fluoroscopy during implantation, but loses its radiopacity after implantation to allow for subsequent imaging under more sensitive radiologic imaging modalities.

The present invention provides a medical device comprising radiopaque water-dispersible metallic nanoparticles, wherein the nanoparticles are released from the medical device upon implantation of the device. The nanoparticles are formed of a metallic material and have surface modifications that impart water-dispersibility to the nanoparticles. The nanoparticles may be any of the various types of radiopaque water-dispersible metallic nanoparticles that are known in the art. The nanoparticles may be adapted to facilitate clearance through renal filtration or biliary excretion. The nanoparticles may be adapted to improve biocompatibility, reduce tissue accumulation, and have reduced toxicity in the human body. The nanoparticles may be applied directly onto the medical device, e.g., as a coating, or be carried on the surface of or within a carrier coating on the medical device, or be dispersed within the pores of a porous layer or porous surface on the medical device. The medical device itself may be biodegradable and may have the nanoparticles embedded within the medical device itself or applied as or within a coating on the biodegradable medical device. The nanoparticles may be released by diffusion through the carrier coating, disruption of hydrogen bonds between the nanoparticles and the carrier coating, degradation of the nanoparticle coating, degradation of the carrier coating, diffusion of the nanoparticles from the medical device, or degradation of the medical device carrying the nanoparticles.

Also provided is a method for providing temporary radiopacity to a medical device, comprising the steps of: (a) providing a medical device; and (b) applying a coating of water-dispersible metallic nanoparticles onto the medical device; wherein the nanoparticles are released from the medical device upon implantation of the medical device.

### BRIEF DESCRIPTION OF THE DRAWINGS

FIGS. 1(a) and 1(b) are images of a stented coronary artery obtained by CT angiography.

FIG. 1(c) is an image of the stented artery of FIGS. 1(a) and 1(b) obtained by contrast dye injection and x-ray fluoroscopy (with the black arrows indicating the stented segment).

FIGS. 2(a)-(c) are images of another stented coronary artery obtained by CT angiography.

FIG. 2(d) is an image of the stented artery of FIGS. 2(a)-(c) obtained by contrast dye injection and x-ray fluoroscopy (with the white arrow indicating the stented segment).

The images shown in FIGS. 1 and 2 were obtained from Maintz et al., Assessment of Coronary Arterial Stents by Multislice-CT Angiography, Acta Radiologica 44:597-603 (2003).

### DETAILED DESCRIPTION

The present invention provides a medical device comprising radiopaque water-dispersible metallic nanoparticles, wherein the nanoparticles are released from the medical device upon implantation of the device.

The term "metallic nanoparticle" refers to a particle having a diameter in the range of about 1 nm to 1000 nm that comprises a metallic material, an alloy, or other mixture of metallic materials. The metallic material may be any metal having sufficient radiopacity for visualization under x-ray fluoroscopy, including iodine, barium, tantalum, tungsten, rhenium, osmium, iridium, noble metals, platinum, gold, and bismuth. Oxides and compounds of the metals listed, such as bismuth subcarbonate and bismuth oxychloride, may also be used. Salts of the metals listed, such as barium salts, iodine salts, or bismuth salts, may also be used.

The term "water-dispersible" refers to the ability of the material to form an essentially unaggregated dispersion of discrete particles or ions that can be sustained indefinitely in an aqueous medium at physiologic temperatures. The term "water-dispersible" is intended to include the ability of a material to form solutions, colloid suspensions, or colloid dispersions in water.

The term "water-dispersible metallic nanoparticle" refers to the aforementioned metallic nanoparticle having surface modifications which impart water-dispersibility to the nanoparticle. Water-dispersible metallic nanoparticles having various types of surface modifications are known in the art. Water-dispersible gold, silver, copper, platinum, and palladium nanoparticles having a layer of organic compounds with reactive functional groups, including thiol, disulfide, sulfide, thiosulfate, xanthate, ammonium, amine, phosphine, phosphine oxide, carboxylate, selenide, and isocyanide groups are described in Shon et al., Metal Nanoparticles Protected with Monolayers: Synthetic Methods, in Dekker Encyclopedia of Nanoscience and Nanotechnology (James A. Schwarz et al. eds., 2004). Water-dispersible amine-stabilized aqueous colloidal gold nanoparticles made using multifunctional oleyl amines are described in Aslam et al., Novel One-Step Synthesis of Amine-Stabilized Aqueous Colloidal Gold Nanoparticles, J. of Materials Chemistry 14:1795-1797 (2004). Water-dispersible gold nanoparticles capped with sodium dodecylsulphate (SDS) and octadecylamine (ODA) are described in Swami et al., Water-Dispersible Nanoparticles Via Interdigitation of Sodium Dodecylsulphate Molecules in Octadecylamine-Capped Gold Nanoparticles at a Liquid-Liquid Surface, Proc. Indian Acad. Sci. 115:679-687 (2003). Water-soluble polymer-coated iron oxide nanoparticles are described in U.S. Patent Publication No. 2003/0124193 (Goldshtein). Water-soluble micelle-encapsulated metal nanoparticle complexes are described in U.S. Patent Publication No. 2004/0033345 (Dubertret et al.). Certain water-dispersible colloidal gold nanoparticles, such as auranofin, aurothioglucose, or gold sodium thiomalate, are used pharmacologically in the treatment of inflammatory or rheumatologic diseases. Soluble metal oxides and mixed metal (doped) oxides, such as titanium oxide, iridium oxide, or tin oxide, can be used to form nanoparticles as described in WO 2005/049520 (Cunningham et al.). Water-dispersible metallic nanoparticles can also be formed by coating a metallic nanoparticle with compositions of soluble metal and mixed metal oxides, such as the compositions described in *Cunningham.* The soluble metal oxides could also complex to the metallic nanoparticles (such as gold nanoparticles) by coordination via the functional groups on the soluble metal oxides. The soluble mixed metal oxides may also be doped with heavier metals, such as platinum or gold, to enhance radiopacity.

Radiopaque coatings formed of water-dispersible nanoparticles are more biocompatible than coatings formed of non-water-dispersible nanoparticles, such as the radiopaque coating of naked metallic nanoparticles described in U.S. Patent No. 6,355,058 (Pacetti et al.).

In some embodiments, the water-dispersible nanoparticles used in the present invention may be adapted to facilitate clearance through renal filtration. The pores of renal glomerular membranes are believed to be about 8 nm (80 angstroms) wide and dextran particles of up to about 42 angstroms have been demonstrated to be filtered through the glomerulus. *See* Arthur C. Guyton & John E. Hall, Textbook of Medical Physiology 284-286 (10th ed. 2000). In addition to the size of the nanoparticles, the charge and surface characteristics of the nanoparticles will affect renal filtration. *See id.* For example, neutral or positively charged nanoparticles are filtered more readily than negatively charged nanoparticles. Thus, one of ordinary skill in the art can select for nanoparticles having the desired characteristics to improve clearance through renal filtration.

In some embodiments, the nanoparticles may be adapted to facilitate clearance through biliary excretion. The mononuclear phagocytic system (MPS), which includes the Kupffer cells in the liver, is involved in the liver uptake and subsequent biliary excretion of nanoparticles. Certain size and surface properties of nanoparticles are known to increase uptake by the MPS in the liver. *See* Choi et al., Surface Modification of Functional Nanoparticles for Controlled Drug Delivery, J. of Dispersion Sci. Tech. 24(3/4):475-487 (2003); *and* Brannon-Peppas et al., Nanoparticles for Delivery of Pifithrins to Combat Cell Death Due to Chemotherapy and Radiation, J. Drug Delivery Sci. Tech. 14(4):257-264 (2004). For example, increasing the hydrophobicity of nanoparticles is known to increase uptake by the MPS. Thus, one of ordinary skill in the art can select for nanoparticles having the desired characteristics to improve biliary excretion.

In some embodiments, the nanoparticles may be adapted to have reduced toxicity in the human body. Characteristics ofnanoparticles that are believed to be factors in determining toxicity include its size, agglomeration state, shape, crystal structure, chemical composition (including spatially averaged (bulk) and spatially resolved heterogeneous composition), surface area, surface chemistry, surface charge, and porosity. *See* Oberdorster et al., Principles for Characterizing the Potential. Human Health Effects From Exposure to Nanomaterials: Elements of a Screening Strategy, Particle and Fibre Toxicology 2:8 (Oct. 6, 2005). Furthermore, the size, hydrophilicity, and surface charge of nanoparticles have been demonstrated to be factors in determining tissue accumulation. *See* Kosar et al., Nanoparticles Administered to the Human Body: Impacts and Implications, News From the Bottom (2004). For example, nanoparticles having a size less than 100 run and having hydrophilic surface modifications are believed to reduce tissue accumulation by avoiding uptake by the reticuloendothelial system (RES) and are believed to allow the nanoparticles to remain in the blood circulation instead of being extravasated through capillary walls. *See* Choi et al., Surface Modification of Functional Nanoparticles for Controlled Drug Delivery, J. of Dispersion Sci. Tech. 24(3/4):475-487 (2003). Thus, one of ordinary skill in the art can select for nanoparticles having the desired characteristics to reduce its toxicity in the human body and/or to reduce its accumulation in body tissue.

The water-dispersible metallic nanoparticles maybe applied onto the medical device in various ways. In an embodiment, the nanoparticles are deposited directly onto the surface of the medical device. Various techniques are available for the deposition of nanoparticles onto substrates, such as chemical vapor deposition, physical vapor deposition, electron beam evaporation, electroplating, or reactive sputtering. Nanoparticles may also be deposited by applying a nanoparticle mixture, such as a solution, sol, sol-gel, or solvent dispersion, onto the substrate and then evaporating of the mixture. Upon implantation of the medical device, interaction with a physiologic environment will cause the nanoparticle coating to break down or degrade by chemical processes such as hydrolysis, dissolution, or corrosion; or physical processes such as abrasion or fluid turbulence.

In another embodiment, the medical device comprises a carrier coating, wherein the nanoparticles are carried on the surface of the carrier coating. The carrier coating may be formed of polymeric materials, which may or may not be biodegradable, such as the polymeric materials that are conventionally used to coat medical devices. Within certain embodiments, the nanoparticles are carried on the surface of a polymer coating and attached thereon via hydrogen bonds between the functional groups on the surface of the nanoparticles and the functional groups on the polymer. Upon implantation and exposure to an aqueous environment, water molecules will disrupt the hydrogen bonds and liberate the nanoparticles from the polymer coating. The hydrogen bonding strength between the polymer coating and the nanoparticles is one of the factors determining the rate at which the nanoparticles are released from the coating. Thus, one of skill in the art can select for nanoparticles or polymer coatings having the desired characteristics to vary the release rate. For example, using polymers that are rich in hydrogen bonding sites, such as polyalkyl-methacrylates, polyethylene-glycols, and polyhydroxy-acids such as polyhydroxy-valerate or polyhydroxy-buterate, would slow the nanoparticle release rate.

In yet another embodiment, the medical device comprises a carrier coating, wherein the nanoparticles are dispersed within the carrier coating. The nanoparticles may be dispersed within the carrier coating using various methods. In some cases, a mixture of the nanoparticles and the carrier coating material is applied onto the medical device by various coating techniques such as spraying, dipping, brushing, electrostatic spraying, or powder coating. In other cases, the carrier coating material is applied first, and then the nanoparticles are embedded into the carrier coating by transfer techniques such as vacuum impregnation or electrophoretic transfer. In other cases, the nanoparticles are applied first to the medical device, and then the carrier coating material is applied over the nanoparticles.

In certain embodiments, the nanoparticles are dispersed within a carrier coating formed of a polymeric material. Upon implantation and exposure to an aqueous environment, the nanoparticles are released by diffusion through the polymer matrix of the coating. One of ordinary skill in the art can vary the diffusion rate of the nanoparticles by altering various characteristics of the polymer coating, such as its composition, porosity, hydrophilicity, or thickness. Within certain embodiments, the carrier coating may be formed of a biodegradable polymer. Upon implantation, the biodegradable polymer coating is degraded by exposure to a physiologic environment, releasing the embedded nanoparticles.

In certain embodiments, the nanoparticles are dispersed within a carrier coating formed of a porous material. Upon implantation and exposure to an aqueous environment, the nanoparticles are released by diffusion through the porous matrix of the carrier coating. The porous material may be any of the various types of porous materials known in the act. Within certain embodiments, the nanoparticles are dispersed within a porous metallic or metallic oxide layer, which may be applied onto the medical device by various coating or deposition methods known in the art, such as electroplating, spray coating, dip coating, sputtering, chemical vapor deposition, or physical vapor deposition. Within certain embodiments, the nanoparticles are dispersed within a porous carbon layer on the medical device, such as the porous carbon layer formed by carbonization as described in U.S. Patent Publication No. 2005/0079200 (Rathenow et al.).

In yet another embodiment, the medical device may comprise a porous surface on the medical device, which may be created by treating the surface of medical device body with micro-roughening processes such as reactive plasma treatment, ion bombardment, or micro-etching. The nanoparticles are dispersed within the porous surface and diffuse out of the porous surface upon implantation of the medical device and exposure to an aqueous environment.

In yet another embodiment, the medical device itself may be biodegradable and may have the nanoparticles embedded within the medical device itself or applied as or within a coating on the biodegradable medical device. The nanoparticles may be released as described above or may be released through diffusion of the nanoparticles from the medical device or degradation of the medical device carrying the nanoparticles.

The medical device of the present invention can be any implantable medical device in which x-ray visualization is desired during implantation, while allowing subsequent follow-up visualization using more sensitive imaging modalities such as CT or MRI. Such medical devices include stents, stent grafts, catheters, guide wires, balloons, filters (e.g., vena cava filters), vascular grafts, intraluminal paving systems, pacemakers, electrodes, leads, defibrillators, joint and bone implants, spinal implants, access ports, intra-aortic balloon pumps, heart valves, sutures, artificial hearts, neurological stimulators, cochlear implants, retinal implants, and other devices that can be used in connection with therapeutic coatings. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like.

Such medical devices may be made of any type of material that is of sufficiently low radiopacity for compatibility with sensitive imaging modalities such as CT or MRI. Such materials include polymers (whether synthetic, natural, biodegradable, or non-biodegradable), amorphous and/or (partially) crystalline carbon, complete carbon material, porous carbon, graphite, composite carbon materials, carbon fibres, ceramics such as zeolites, silicates, aluminium oxides, aluminosilicates, silicon carbide, silicon nitride; metals such as titanium, zircon, vanadium, chromium, molybdenum, manganese, cobalt, nickel, copper, and alloys, carbides, oxides, nitrides, carbonitrides, oxycarbides, oxynitrides, and oxycarbonitrides of such metals; shape memory alloys such as nitinol, nickel-titanium alloys, glass, stone, glass fibres, minerals, natural or synthetic bone substance bone, imitates based on alkaline earth metal carbonates such as calcium carbonate, magnesium carbonate, strontium carbonate and any desired combinations of the above-mentioned materials.

The polymeric materials used in the medical device of the present invention may be biodegradable or non-biodegradable. Non-limiting examples of suitable non-biodegradable polymers include polystyrene; polyisobutylene copolymers such as styrene-isobutylene-styrene (SIBS) block copolymers and styrene-ethytene/butylene-styrene (SEBS) block copolymers; polyvinylpyrrolidone including cross-linked polyvinylpyrrolidone; polyvinyl alcohols, copolymers of vinyl monomers such as EVA; polyvinyl ethers; polyvinyl aromatics; polyethylene oxides; polyesters including polyethylene terephthalate; polyamides; polyacrylamides including poly(methylmethacrylate-butylacetate-methylmethacrylate) triblock copoylmers; polyethers including polyether sulfone; polyalkylenes including polypropylene, polyethylene and high molecular weight polyethylene; polyurethanes; polycarbonates, silicones; siloxane polymers; cellulosic polymers such as cellulose acetate; polymer dispersions such as polyurethane dispersions (BAYHYDROL®); squalene emulsions; and mixtures and copolymers of any of the foregoing.

Non-limiting examples of suitable biodegradable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L-lactide), poly(lactic acid-co-glycolic acid), 50/50 (DL-lactide-co-glycolide); polydioxanone; polypropylene fumarate; polydepsipeptides; polycaprolactone and co-polymers and mixtures thereof such as poly(D.L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate.

The medical device of the present invention may also comprise a therapeutic agent, which may be dispersed within the carrier coating or within another coating on the medical device to provide controlled release.

## Claims

1. A medical device comprising water dispersible radiopaque metallic nanoparticles having a size less than 100 nm, wherein the nanoparticles have hydrophilic surface modifications, and wherein the nanoparticles are released from the medical device upon implantation of the medical device into a patient.

2. The medical device of claim 1, wherein the surface modifications comprise water-soluble functional groups.

3. The medical device of claim 1, wherein the surface modifications comprise hydrophilic functional groups.

4. The medical device of claim 1, wherein the surface modifications comprise organic molecules complexed to the surface of the nanoparticles.

5. The medical device of claim 1, wherein the surface modifications comprise soluble metal oxides or soluble mixed metal oxides.

6. The medical device of claim 1, wherein the medical device further comprises a carrier coating.

7. The medical device of claim 6, wherein the carrier coating comprises a polymer.

8. The medical device of claim 7, wherein the polymer is a biodegradable polymer.

9. The medical device of claim 7, wherein the carrier coating comprises a porous material.

10. The medical device of claim 9, wherein the porous material is a porous carbon material.

11. The medical device of claim 1, wherein the medical device further comprises a porous surface, and wherein the nanoparticles are dispersed within the pores of the porous surface.

12. The medical device of claim 1, wherein the medical device is biodegradable.

13. The medical device of claim 1, wherein the nanoparticles have a neutral or positive electrostatic charge.

## Patentansprüche

1. Eine medizinische Vorrichtung umfassend wasserdispersible, radiopaque, metallische Nanopartikel mit einer Größe von weniger als 100 nm, wobei die Nanopartikel hydrophile Oberflächenmodifikationen aufweisen, und wobei die Nanopartikel nach einer Implantation der medizinischen Vorrichtung in einen Patienten von der medizinischen Vorrichtung freigegeben werden.

2. Medizinische Vorrichtung nach Anspruch 1, wobei die Oberflächenmodifikationen wasserlösliche funktionelle Gruppen umfassen.

3. Medizinische Vorrichtung nach Anspruch 1, wobei die Oberflächenmodifikationen hydrophile funktionelle Gruppen umfassen.

4. Medizinische Vorrichtung nach Anspruch 1, wobei die Oberflächenmodifikationen organische Moleküle umfassen, die an die Oberfläche der Nanopartikel komplexiert sind.

5. Medizinische Vorrichtung nach Anspruch 1, wobei die Oberflächenmodifikationen lösliche Metalloxide oder lösliche Mischmetalloxide umfassen.

6. Medizinische Vorrichtung nach Anspruch 1, wobei die medizinische Vorrichtung weiterhin eine Trägerbeschichtung umfasst.

7. Medizinische Vorrichtung nach Anspruch 6, wobei die Trägerbeschichtung ein Polymer umfasst.

8. Medizinische Vorrichtung nach Anspruch 7, wobei das Polymer ein biologisch abbaubares Polymer ist.

9. Medizinische Vorrichtung nach Anspruch 7, wobei die Trägerbeschichtung ein poröses Material umfasst.

10. Medizinische Vorrichtung nach Anspruch 9, wobei das poröse Material ein poröses Kohlenstoffmaterial ist.

11. Medizinische Vorrichtung nach Anspruch 1, wobei die medizinische Vorrichtung weiterhin eine poröse Oberfläche umfasst und wobei die Nanopartikel innerhalb der Poren der porösen Oberfläche verteilt sind.

12. Medizinische Vorrichtung nach Anspruch 1, wobei die medizinische Vorrichtung biologisch abbaubar ist.

13. Medizinische Vorrichtung nach Anspruch 1, wobei die Nanopartikel eine neutrale oder positive elektrostatische Ladung aufweisen.

## Revendications

1. Un dispositif médical comprenant des nanoparticules métalliques radio-opaques dispersibles dans l'eau présentant une dimension inférieure à 100 nm, où les nanoparticules présentent des modifications de surface hydrophiles et où les nanoparticules sont libérées du dispositif médical à l'implantation du dispositif médical chez un patient.

2. Le dispositif médical de la revendication 1, dans lequel les modifications de surface comprennent des groupes fonctionnels solubles dans l'eau.

3. Le dispositif médical de la revendication 1, dans lequel les modifications de surface comprennent des groupes fonctionnels hydrophiles.

4. Le dispositif médical de la revendication 1, dans lequel les modifications de surface comprennent des molécules organiques complexées à la surface des nanoparticules.

5. Le dispositif médical de la revendication 1, dans lequel les modifications de surface comprennent des oxydes métalliques solubles ou des oxydes métalliques mélangés solubles.

6. Le dispositif médical de la revendication 1, dans lequel le dispositif médical comprend en outre un revêtement porteur.

7. Le dispositif médical de la revendication 6, dans lequel le revêtement porteur comprend un polymère.

8. Le dispositif médical de la revendication 7, dans lequel le polymère est un polymère biodégradable.

9. Le dispositif médical de la revendication 7, dans lequel le revêtement porteur comprend un matériau poreux.

10. Le dispositif médical de la revendication 9, dans lequel le matériau poreux est un matériau de carbone poreux.

11. Le dispositif médical de la revendication 1, dans lequel le dispositif médical comprend en outre une surface poreuse, et où les nanoparticules sont dispersées au sein des pores de la surface poreuse.

12. Le dispositif médical de la revendication 1, dans lequel le dispositif médical est biodégradable.

13. Le dispositif médical de la revendication 1, dans lequel les nanoparticules présentent une charge électrostatique neutre ou positive.
